# EUROPÄISCHE PATENTSCHRIFT

(11) **EP 3 062 830 B1**
(45) Veröffentlichungstag und Bekanntmachung des Hinweises auf die Patenterteilung: **06.12.2017**
(21) Anmeldenummer: 14793503.5
(22) Anmeldetag: 30.10.2014
(51) Int. Cl.: A61L 15/18, A61L 15/44, A61L 26/00

(54) **BLUTSTILLENDES MITTEL ENTHALTEND KRISTALLINES POLYPHOSPHAT**
HAEMOSTATIC COMPOSITION COMPRISING CRYSTALLINE POLYPHOSPHATE
PRODUIT HÉMOSTATIQUE CONTENANT UN POLYPHOSPHATE CRISTALLIN

(30) Priorität: 31.10.2013 DE 102013222223
(43) Veröffentlichungstag der Anmeldung: 07.09.2016
(73) Patentinhaber: BK Giulini GmbH, 67065 Ludwigshafen (DE)
(72) Erfinder: THAUERN, Henrike, 69469 Weinheim (DE); STAFFEL, Thomas, 67269 Grünstadt (DE)
(74) Vertreter: Reitstötter Kinzebach
(86) Internationale Anmeldenummer: PCT/EP2014/073274
(87) Internationale Veröffentlichungsnummer: WO 2015/063190

(56) Entgegenhaltungen:
- US-A- 2 852 341
- US-A1- 2006 198 837
- US-A1- 2007 154 510

## Beschreibung

Die vorliegende Erfindung betrifft Polyphosphat in kristalliner Form zur Verwendung in der Behandlung blutender Wunden, Mittel enthaltend kristallines Polyphosphat sowie Verfahren zur Herstellung solcher Mittel.
Durch Verletzungen von menschlichen und tierischen Gewebeschichten können darin befindliche Blutgefäße beschädigt werden, was zu Blutaustritt an der betroffenen Stelle auf der Haut oder im Körperinneren führen kann. Nach einer Phase des Blutaustritts setzt die natürliche Blutgerinnung ein. Die Blutgerinnung bezeichnet das Erstarren des flüssigen Blutes als physiologischen Schutzmechanismus. Die Blutgerinnung basiert auf zwei komplexen, kaskadenartig ablaufenden enzymatischen Reaktionswegen, an denen mehrere Gerinnungsfaktoren mitwirken. Beide Wege, die üblicherweise als intrinsischer und extrinsischer Weg bezeichnet werden, münden in die Bildung unlöslicher Fibrinfäden, die maßgeblich am Zustandekommen von wundschließenden Blutgerinnseln beteiligt sind. Thrombin, auch Gerinnungsfaktor IIa genannt, nimmt hierbei eine Schlüsselstellung ein, da es nicht nur die Bildung von Fibrin bewirkt, sondern auch durch positive Rückkopplung an mehren Stellen der Gerinnungskaskade aktivierend eingreift.

Die natürliche Blutgerinnung setzt zwar fast unmittelbar nach Eintritt des Traumas ein, ist aber ein relativ langsamer Prozess, der zum Verschließen der Wunde und damit einhergehender Blutstillung einige Zeit beansprucht. In vielen Fällen ist daher eine beschleunigte Blutstillung wünschenswert, beispielsweise aus hygienischen Gründen oder wenn aufgrund der Größe der Wunde oder der Stärke des Blutflusses ein übermäßiger Blutverlust zu befürchten ist. Altbekannte Methoden zur Blutstillung, wie etwa Abdecken der Wunde, Anlegen von Druckverbänden und Klammern oder Nähen der Wunde, sind oftmals unzureichend. Aus dem Stand der Technik sind daher Verfahren bekannt, die zur Verbesserung der Blutstillung beitragen sollen, beispielsweise das Einbringen blutgerinnungsaktiver Substanzen wie Fibrinkleber in den Wundbereich. Verwendungen von anorganischen Substanzen als blutstillende Mittel wurden in jüngerer Zeit beispielsweise in den folgenden Patentschriften beschrieben:
US 2007/0160653 betrifft Glasfasern enthaltende textile Gewebe, die die körpereigene Blutstillung aktivieren.
Die in US 2006/0039994 beschriebenen Methoden zur Förderung der Blutgerinnung beruhen auf der Wirkung von Molekularsieben auf Basis von Aluminiumphosphat.
In US 2008/0145447 und US 2009/0047366 werden Kieselgur, Kieselgel, Glasfasern, Quartzsand, Calciumsilikat und amorphes Calciumpolyphosphat als die Blutgerinnung anregende Stoffe beschrieben.

Die aktivierenden Einflüsse von wasserlöslichen Polyphosphaten auf die Blutgerinnung werden von S.A. Smith et al., PNAS 2006, 103(4), 903-908; S.A. Smith et al., J. Thromb. Haemost. 2008, 6(10), 1750-1756; S.A. Smith et al., Blood 2008, 112, 2810-2816; F. Müller et al., Cell 2009, 139, 1143-1156; und S.A. Smith et al., Blood 2010, 116, 4353-4359 diskutiert. Darüberhinaus beschreibt EP 1 869 082 die Verwendung von wasserlöslichem Polyphosphat mit einer Kettenlänge von mindestens 25 zum Stillen oder Verlangsamen einer Wundblutung. US 2007/154510 A offenbart einen hämostatischen Artikel umfassend einen porösen Träger und Polyphosphatglas (Natriumhexametaphosphat) zur effektiven Blutstillung.

Bei den aus dem Stand der Technik bekannten partikulären Stoffen zur Blutstillung handelt es sich in der Mehrzahl um unphysiologische Substanzen, deren Verträglichkeit für den menschlichen oder tierischen Organismus beim Kontakt mit Wundgewebe häufig zweifelhaft ist und die möglicherweise allergieauslösend sind. Außerdem ist für viele der genannten partikulären Stoffe nicht gewährleistet, dass sie frei von potentiell toxischen oder anderweitig unverträglichen Verunreinigungen sind. Auch das beispielsweise in der EP 1869 082 verwendete vollständig wasserlösliche Polyphosphat ist mit Nachteilen behaftet. So wird es bei starken Blutungen schnell aus dem Wundgebiet weggeschwemmt und somit unwirksam, während es bei schwächeren Blutungen leicht in die Blutbahn gelangen kann und unerwünschte Auswirkungen an von der Wunde weit entfernten Stellen des Körpers haben kann, wie beispielweise die Bildung von thrombotischen Blutgerinnseln.
Die Aufgabe der Erfindung liegt daher in der Bereitstellung einer physiologisch verträglichen blutstillenden Substanz und eines entsprechenden Mittels für die Wundbehandlung, welche die Probleme der Produkte des Standes der Technik überwinden, sich einfach und kostengünstig herstellen lassen und in der medizinischen Praxis mittels üblicher Methoden leicht und zuverlässig anwendbar sind. Überraschenderweise wurde gefunden, dass die gestellte Aufgabe gelöst wird durch die Behandlung von Wunden mit Polyphosphat in kristalliner Form, wobei das Anion des Polyphosphats im Mittel (Zahlenmittel) mindestens 4 Phosphoratome pro Polyphosphat-Anion aufweist.

Die genannten kristallinen Polyphosphate besitzen nicht nur eine sehr gute blutstillende Wirkung, sondern sind auch leicht zu handhaben, physiologisch verträglich und lassen sich kostengünstig in hochreiner Form herstellen.

Gegenstand der Erfindung ist daher Polyphosphat in kristalliner Form zur Verwendung in der Behandlung von Wunden, insbesondere blutender Wunden, wobei das Anion des Polyphosphats im Mittel (Zahlenmittel) mindestens 4 Phosphoratome pro Polyphosphat-Anion aufweist.
Ein weiterer Gegenstand der Erfindung sind Mittel, die ein in kristalliner Form vorliegendes Polyphosphat gemäß der Erfindung und ein Trägermaterial umfassen. Diese Mittel sind zur Behandlung von Wunden, insbesondere blutenden Wunden, geeignet.
Ein weiterer Gegenstand der Erfindung sind Verfahren zur Herstellung des erfindungsgemäßen Mittels, die das Einbringen des Polyphosphats und gegebenenfalls weiterer blutstillender Agenzien in das Trägermaterial, bzw. in eine Komponente oder in eine Vorstufe des Trägermaterials, umfassen.
Die kristallinen Polyphosphate gemäß der Erfindung weisen in der Regel einen Kristallinitätsgrad von mindestens 90%, vorzugsweise mindestens 95% und insbesondere mindestens 98% auf. Der Kristallinitätsgrad des Polyphosphats kann in an sich bekannter Weise mittels Röntgen-Pulverdiffraktometrie oder durch DSC-Messungen bestimmt werden.

Die Anionen der kristallinen Polyphosphate der Erfindung sind in der Regel im wesentlichen, d. h. zu wenigstens 80 Mol-%, bevorzugt wenigstens 90 Mol-% und insbesondere wenigstens 95 Mol-%, bezogen auf das Polyphosphat, linear.
Bei den Kationen der kristallinen Polyphosphate handelt es sich bevorzugt um Alkalimetall-, Erdalkalimetall-, Metall- oder Ammoniumionen, die unabhängig voneinander insbesondere unter Na⁺, K⁺, NH₄⁺, Ca²⁺, Mg²⁺, Zn²⁺, Sn²⁺, Fe³⁺ und Al³⁺, vorzugsweise unter Na⁺, K⁺, NH₄⁺ und Ca²⁺, ausgewählt sind.
In einer bevorzugten Ausführungsform der Erfindung weisen die Polyphosphat-Anionen der erfindungsgemäßen Polyphosphate im Mittel (Zahlenmittel) 8 bis 500, vorzugsweise 12 bis 300 und insbesondere 15 bis 150 Phosphoratome pro Polyphosphat-Anion auf. Die zahlenmittlere Anzahl der Phosphoratome im Polyphosphat-Anion kann in an sich bekannter Weise z. B. durch ³¹P-Festkörper-NMR bestimmt werden, beispielsweise mittels der nachstehend näher beschriebenen Vorgehensweise.

Im Kontext der vorliegenden Erfindung steht "lineare Polyphosphate" für die Salze nicht-zyklischer Polyphosphat-Anionen. Somit handelt es sich bei den linearen Polyphosphaten der Erfindung um Ketten aus meta-Phosphateinheiten PO₃⁻, in denen jeweils zwei Phosphoratome über ein Sauerstoffatom miteinander verbunden sind. Entsprechend wird hier unter "Kettenlänge" die Anzahl der im Polyphosphat-Anion enthaltenen meta-Phosphateinheiten PO₃⁻ verstanden. Die linearen kristallinen Polyphosphate der Erfindung können durch die Formel A beschrieben werden: 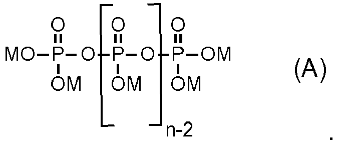 In der Formel A steht n im Mittel (Zahlenmittel) für eine Zahl von 4 bis 1000, vorzugsweise von 8 bis 500, insbesondere von 12 bis 300 und speziell von 15 bis 150. M steht für ein Kation bzw. ein Kationenäquivalent, das jeweils unabhängig vorzugsweise unter Na⁺, K⁺, ½Ca²⁺ und NH₄⁺ ausgewählt ist. Somit entspricht die Variabel n in der Formel A der zahlenmittleren Kettenlänge des linearen Polyphosphats bzw. der zahlenmittleren Anzahl der Phosphoratome des Polyphosphat-Anions. Der Ausdruck "½Ca²⁺" verdeutlicht hier, dass jedes Ca²⁺, welches in dem Polyphosphat der Formel A enthalten ist, aufgrund seiner zweifach positiven Ladung für zwei Kationen M in der Formel A steht.

Die erfindungsgemäß verwendeten kristallinen Polyphosphate zeichnen sich durch hohe Reinheiten aus. So beträgt der Anteil an Wasser, das als Restfeuchte noch im kristallinen Polyphosphat enthalten sein kein, üblicherweise weniger als 1 Gew.-%, vorzugsweise weniger als 0,5 Gew.-% und insbesondere weniger als 0,3 Gew.-%, bezogen auf das Gesamtgewicht des Polyphosphats. Der Anteil an Inhaltsstoffen, bei denen es sich nicht um Phosphate oder Wasser handelt, liegt in der Regel bei weniger als 0,1 Gew.-%, bevorzugt weniger als 0,05 Gew.-% und insbesondere weniger als 0,01 Gew.-%, in Bezug auf das Gesamtgewicht des Polyphophats. In diesem Zusammenhang werden unter Phosphaten Mono-, Meta-, Pyro-, Tri- und Polyphosphate verstanden. Darüber hinaus ist der Anteil an Mono-, Meta-, Pyro- und Triphosphaten sowie Polyphosphaten deren Kettenlänge wenigstens 20% von der zahlenmittleren Kettenlänge des jeweiligen erfindungsgemäßen Polyphosphats abweicht in der Regel kleiner als 10 Gew.-%, bevorzugt kleiner als 7,5 Gew.-% und insbesondere kleiner als 5 Gew.-%, in Bezug auf das Gesamtgewicht des Polyphosphats.

Die erfindungsgemäß verwendeten kristallinen Polyphosphate zeichnen sich des Weiteren dadurch aus, dass sie im Wesentlichen wasserunlöslich sind. So beträgt ihr Anteil an wasserlöslichen Bestandteilen in der Regel weniger als 8 Gew.-%, vorzugsweise weniger als 5 Gew.-% und insbesondere weniger als 3 Gew.-%, in Bezug auf das Gesamtgewicht des Polyphophats. Als wasserlöslich werden hier solche Bestandteile bezeichnet, die sich in Form eines Pulvers bei 25°C mit einer Rate von mindestens 50 Gew.-% pro Stunde in Wasser auflösen. Bei solchen wasserlöslichen Bestandteilen handelt es sich praktisch ausschließlich um die vorgenannten Mono-, Meta-, Pyro- und Triphosphate, die eine hohe Wasserlöslichkeit besitzen.

In einer bevorzugten Ausführungsform der Erfindung sind alle Kationen des erfindungsgemäßen Polyphosphats unter Na⁺, K⁺, Ca²⁺ und NH₄⁺ und insbesondere unter Na⁺, K⁺ und NH₄⁺ ausgewählt. Besonders bevorzugt sind alle Kationen des Polyphosphats entweder Na⁺, K⁺, Ca²⁺ oder NH₄⁺ und insbesondere Na⁺, K⁺ oder NH₄⁺, d. h., das Polyphosphat enthält keine unterschiedlichen Kationen.

Die in kristalliner Form vorliegenden Polyphosphate gemäß der vorliegenden Erfindung lassen sich mit aus dem Stand der Technik bekannten Verfahren, die beispielsweise von K. Schrödter et al. in: Ullmann's Encyclopedia of Industrial Chemistry, "Phosphoric Acid and Phosphates", Seiten 32 - 41, 2008 (7. Auflage), New York, NY, Wiley-VCH Verlag, beschrieben sind, herstellen. So können kristalline Natriumpolyphosphate wie das Kurrolsche Salz und das Maddrellsche Salz beispielsweise durch Tempern von amorphen, glasartigem Natriumpolyphosphat hergestellt werden, welches seinerseits hergestellt werden kann, indem man Natriumdihydrogenphosphat und Natriumhydrogenphosphat unter Freisetzung des Kondensationswassers erhitzt und die erhaltene Schmelze anschließend abschreckt. Kristalline Kalium- und Calciumpolyphosphate lassen sich in ähnlicher Weise, beispielsweise durch Erhitzen von KH₂PO₄ bzw. Ca(H₂PO₄)₂ herstellen. Dagegen können kristalline Ammoniumpolyphosphate zum Beispiel hergestellt werden, indem man Phosphorsäure mit Ammoniak freisetzenden Verbindungen, wie etwa Harnstoff, Melamin oder Urotropin, umgesetzt, oder alternativ Phosphorpentoxid mit Diammoniumphosphat in Ammoniak umsetzt.

In einer besonders bevorzugten Ausführungsform der Erfindung sind die in kristalliner Form vorliegenden Polyphosphate ausgewählt unter linearen Natriumpolyphosphaten, d. h. Polyphosphaten der Formel A wobei jedes M für Na⁺ steht, mit einer zahlenmittleren Kettenlänge von 15 bis 100, vorzugsweise 20 bis 80, insbesondere 30 bis 60 und speziell 35 bis 55, linearen Ammoniumpolyphosphaten, d. h. Polyphosphaten der Formel A wobei jedes M für NH₄⁺ steht, mit einer zahlenmittleren Kettenlänge von 12 bis 200, vorzugsweise 17 bis 150, insbesondere 20 bis 135 und speziell 22 bis 125, linearen Calciumpolyphosphaten, d. h. Polyphosphaten der Formel A wobei jedes M für ½ Ca²⁺ steht, mit einer zahlenmittleren Kettenlänge von 8 bis 100, vorzugsweise 10 bis 60, insbesondere 10 bis 40 und speziell 12 bis 25, sowie linearen Kaliumpolyphosphaten, d. h. Polyphosphaten der Formel A wobei jedes M für Ka⁺ steht, mit einer zahlenmittleren Kettenlänge von 12 bis 100, vorzugsweise 18 bis 70, insbesondere 22 bis 50 und speziell 25 bis 40.

Die kristallinen Polyphosphate der Erfindung werden in den erfindungsgemäßen Verwendungen zur Blutstillung und in den erfindungsgemäßen Mitteln zu Wundbehandlung üblicherweise in partikulärer Form eingesetzt. Darunter wird hier verstanden, dass das Polyphosphat zu mindestens 90 Gew.-% aus Partikeln besteht, die in der Regel einen Durchmesser von maximal 5 mm und insbesondere maximal 2 mm haben. Die gewichtsmittlere Teilchengröße der für Zwecke der Blutstillung eingesetzten Polyphosphat liegt in der Regel im Bereich von 0,1 µm bis 5 mm, vorzugsweise 0,5 µm bis 2 mm, insbesondere 1 µm bis 1 mm und speziell 5 µm bis 0,5 mm. Die hier und im Folgenden angegebenen Partikelgrößen können in an sich bekannter Weise mittels Siebanalyse oder durch Lichtstreuung bestimmt werden. Entsprechende partikuläre Polyphosphate mit geeigneter Partikelgrößenverteilung lassen sich mittels Zerkleinern der erfindungsgemäßen kristallinen Polyphosphate in an sich bekannter Weise, insbesondere durch Mahlen, beispielsweise mit einer Kugelmühle, erhalten.

In den erfindungsgemäßen Verwendungen zur Behandlung von blutenden Wunden werden die vorzugweise in partikulärer Form vorliegenden kristallinen Polyphosphate der Erfindung mit dem aus der Wunde austretendem Blut in Kontakt gebracht. Durch den Kontakt mit dem Blut aktiviert das kristalline Polyphosphat einen oder mehrere körpereigene Prozesse, die eine blutstillende Wirkung zur Folge haben, d. h. den Blutfluss verringern, verlangsamen oder stoppen. Bei diesen von dem kristallinen Polyphosphat aktivierten Prozessen handelt es sich vermutlich vorrangig um solche, die von Thrombin beeinflusst und insbesondere aktiviert werden. Hier kommen wahrscheinlich in erste Linie Prozesse in Betracht, die Blutgerinnung durch Bildung von unlöslichem Fibrin bewirken. So ist es beispielsweise vorstellbar, dass die für das intrinsische System bekannte Kontaktaktvierung der Gerinnungskaskade durch das erfindungsgemäße kristalline Polyphosphat ausgelöst wird.

Es wurde gefunden, dass der Kontakt des kristallinen Polyphosphats mit Blut bei den erfindungsgemäßen Verwendungen zu einer Erhöhung des Thrombingehalts im Blut führt. Dabei handelt es sich insbesondere um einen exponentiellen Anstieg des Thrombingehalts.

Die erfindungsgemäße Verwendung von kristallinen Polyphosphaten zur Blutstillung kann so erfolgen, dass das vorzugsweise in partikulärer Form vorliegende Polyphosphat als solches zum Einsatz kommt. Dabei kann beispielsweise partikuläres Polyphosphat in die blutende Wunde eingebracht werden bzw. die Wunde teilweise oder vollständig mit partikulärem Polyphosphat bedeckt werden, so dass ein möglichst intensiver Kontakt zwischen Polyphosphat und Blut gewährleistet ist. Anschließend kann die auf diese Weise behandelte Wunde mittels bekannter Materialen, wie insbesondere Verbandsmaterialien, zum Schutz der Wunde und zum Fixieren der Polyphosphatpartikel abgedeckt bzw. verschlossen werden.

Vorzugsweise erfolgt die erfindungsgemäße Verwendung von kristallinen Polyphosphaten zur Blutstillung jedoch in der Weise, dass die Wunde mit einem erfindungsgemäßen Mittel behandelt wird, welches das kristalline Polyphosphat und ein Trägermaterial umfasst. Dem Trägermaterial kommt dabei insbesondere die Aufgabe zu, das vorzugsweise partikuläre Polyphosphat so zu halten bzw. zu fixieren, dass ein Eindringen des Polyphosphats in tiefer liegende Wundbereiche verhindert wird, gleichzeitig aber ein intensiver Kontakt des Polyphosphatpartikel mit dem austretendem Blut sichergestellt ist.

Die in den erfindungsgemäßen Mitteln enthaltenen Trägermaterialen sind vorzugsweise ausgewählt unter textilen Materialen, Flüssigkeiten, Gelen, Pulvern und Kombinationen daraus, wobei es sich bei diesen Materialien insbesondere um solche handelt, deren Eignung für die Behandlung von Wunden bekannt ist und die für diesen Zweck bereits eingesetzt werden.

Als textile Trägermaterialien eignen sich besonders flächenförmige Gewebe und Vliesstoffe. Die Gewebe und Vliesstoffe sind beispielsweise aus natürlichen Fasern, z.B. Baumwolle, synthetischen Fasern, z.B. Polyester- oder Polyamidfasern, oder Fasermischungen aufgebaut. Diese flächenförmigen Textilien lassen sich in ein- oder mehrlagiger Form in den erfindungsgemäßen Mitteln einsetzen, wobei Lagen aus Gewebe mit solchen aus Vliesstoff auch in Kombination verwendet werden können. Beispiele für geeignete flächenförmige Textilien sind Gaze, Mull und andere flächenförmige Wundauflagen, die Fasern enthalten. Des Weiteren können auch nichtflächenförmige textile Materialien, wie beispielsweise Watten, Faserballen und Tupfer, als Trägermaterial Verwendung finden. Mittel zur Wundbehandlung, die auf solchen textilen Trägermaterialien beruhen, sind zum Beispiel Wundauflagen, Verbandsmaterialien, Binden, Kompressen, Bandagen und Pflaster.

Als flüssige Trägermaterialen sind vor allem hydrophile, insbesondere wässrige, Flüssigkeiten und hydrophobe, insbesondere öl- oder fetthaltige, Flüssigkeiten, sowie Mischungen daraus geeignet. Diese Flüssigkeiten können weitere Substanzen, wie beispielsweise Salze, partikuläre Wirkstoffe oder Gase, in gelöster oder ungelöster Form enthalten. Geeignete Mischungen aus hydrophilen und hydrophoben Flüssigkeiten sind insbesondere Öl-in-Wasser- und Wasser-in-Öl-Emulsionen, die gegebenenfalls emulgierende Substanzen und weitere Inhaltsstoffe enthalten können. Beispiele solcher Flüssigkeiten sind Wasser, wässrige Lösungen, Lotionen, Cremes und Salben. Mittel zur Wundbehandlung, die auf solchen Flüssigkeiten beruhen, sind zum Beispiel Wundcremes, Wundpasten, Wundemulsionen, Wundsprays, bei denen die Flüssigkeit durch ein Treibgas in ein Aerosol überführt wird, und Wundschäume.

Als Trägermaterial geeignete Gele sind insbesondere Hydrogele mit einer festen Phase, die auf natürlichen oder synthetischen Polymeren beruht. Beispiele solcher Polymere sind Polyvinylalkohole, Acrylat-basierte Polymere und Copolymere, verdickend wirkende Polysaccharide wie Agarose, Methylcellulose, Hyaluronsäure, sowie Derivate und Mischungen dieser Polymere. Die wässrige Phase dieser Gele kann weitere Komponenten in gelöster Form enthalten. Mittel zur Wundbehandlung, die auf solchen Gelen beruhen, sind insbesondere Wundgels.

Als pulverförmige Trägermaterial sind beispielsweise feinpartikuläre Mineralien, wie Zeolithe, Kieselgel, Kieselgur oder Kaolin, und feinpartikuläre Polysaccharide, wie Zellulose oder Zellulosederivate, sowie Mischungen aus diesen Materialien geeignet. Diese Pulver können weitere feste Substanzen enthalten, die durch nichtkovalente Bindung an den Puderpartikeln gebunden sind. Zu Mitteln zur Wundbehandlung, die auf solchen Pulvern beruhen, sind insbesondere Wundpuder.

Dementsprechend sind solche der erfindungsgemäßen Mittel bevorzugt, die in Form einer Wundauflage, eines Verbandsmaterials, eines Wundpuders, eines Wundsprays, eines Wundschaums, eines Wundgels, einer Wundcreme oder einer Wundpaste vorliegen.

Weiterhin sind solche der erfindungsgemäßen Mittel bevorzugt, in deren Trägermaterial das kristalline Polyphosphat in partikulärer Form verteilt vorliegt, wobei die Polyphosphatpartikel im gesamten Trägermaterial oder nur in einem Teil davon verteilt sein können. Vorzugsweise sind die Polyphosphatpartikel gleichmäßig oder annähernd gleichmäßig im Trägermaterial oder einem Teil davon verteilt, d.h. das Trägermaterial oder der Teil davon indem sich die Partikel befinden, könnte in gleich große oder annähernd gleich große Segmente unterteilt werden, in denen sich jeweils nur ein Partikel befindet.

In einer Ausführungsform der Erfindung liegt das kristalline Polyphosphat in partikulärer Form verteilt in einer oder mehreren näherungsweise zweidimensionalen Schichten des Trägermaterials vor. Beispielsweise können die Polyphosphatpartikel zwischen zwei Lagen eines flächenförmigen, blutdurchlässigen Trägermaterials, insbesondere eines Gewebes oder eines Vlieses, eingeschlossen sein.

In einer weiteren Ausführungsform der Erfindung liegt das kristalline Polyphosphat in partikulärer Form verteilt in einem dreidimensionalen Trägermaterial oder einem dreidimensionalen Bereich des Trägermaterials vor. Beispielsweise können die Polyphosphatpartikel im Volumen eines Faserballens, Wattebausches oder Gels verteilt sein.

Das erfindungsgemäße Mittel kann gemäß einer Ausführungsform der Erfindung zusätzlich wenigstens ein weiteres blutstillendes Agens enthalten, bei dem es sich vorzugsweise um eine Substanz handelt, deren blutstillende Wirkung aus dem Stand der Technik bekannt ist. Vorzugsweise ist dies zusätzliche blutstillende Agens ausgewählt unter Chitosan, dessen Derivaten, Thrombin, Fibrin, Fibrinogen und Ca²⁺-Ionen enthaltenden Substanzen, die von kristallinem Calciumpolyphosphat verschieden sind, wie beispielsweise Calciumchlorid.

Die erfindungsgemäßen Mittel können weitere Komponenten enthalten, die sich bei Mitteln zur Wundbehandlung als hilfreich erwiesen haben, wie beispielsweise klebende Elemente zur Befestigung auf der Haut oder eine äußere Beschichtung, die die Wunde vor externen Einflüssen schützt bzw. den Austritt von Blut minimiert.

In einer bevorzugten Ausführungsform der Erfindung wird das erfindungsgemäße Mittel zur Wundbehandlung in steriler Form eingesetzt. Demgemäß betrifft die Erfindung auch erfindungsgemäße Mittel, die steril sind, sowie erfindungsgemäße Mittel in steriler Form, die in einer Verpackung eingeschlossen sind, so dass sie auch über längere Lagerperioden ihre Sterilität nicht verlieren.

Bevorzugte Verfahren zur Herstellung der erfindungsgemäßen Mittel für die Behandlung von blutenden Wunden umfassen einen oder mehrere Schritte, die dazu geeignet sind das in partikulärer Form vorliegende kristalline Polyphosphat, sowie gegebenenfalls ein oder mehrere weitere blutstillende Agenzien, in das Trägermaterial, in eine Komponente des Trägermaterials oder in eine Vorstufe des Trägermaterials einzuarbeiten. In diesem Zusammenhang steht "Komponente des Trägermaterials" für einen bestimmten Teil des Trägermaterials, wie beispielsweise eine Gewebelage innerhalb eines mehrlagigen textilen Trägermaterials, oder die flüssige Phase eines aus fester und flüssiger Phase bestehenden Gels. "Vorstufe des Trägermaterials" steht hier für eine Substanz aus der sich durch chemische Umwandlung das Trägermaterial oder ein Teil davon erzeugen lässt, wie beispielsweise ein Monomer, das zur Herstellung eines polymeren Trägermaterials dient.

Die Einarbeitung des kristallinen Polyphosphats in das Trägermaterial, eine Komponente oder eine Vorstufe davon, erfolgt in der Regel indem man das Trägermaterial, die Komponente oder die Vorstufe mit dem Polyphosphat in Kontakt bringt. Falls das Trägermaterial, die Komponente oder die Vorstufe in flüssiger oder partikulärer Form vorliegt, wird die Einarbeitung vorzugsweise durch Vermischen mit den Polyphosphatpartikeln durchgeführt. Auf diese Weise lassen sich Polyphosphatpartikel insbesondere in die Trägermaterialien Puder und Flüssigkeiten einarbeiten, wobei letztere auch Trägermaterialkomponenten beispielsweise einer Emulsion oder eines Gels sein können. Im Falle eines flüssigen oder gelösten Monomers als Trägermaterialvorstufe können die Polyphosphatpartikel mit dem Monomer vermischt werden und anschließend daraus das die Partikel umfassende polymere Trägermaterial durch Polymerisation erhalten werden.

Falls es sich bei dem Trägermaterial, der Komponente oder der Vorstufe davon, um ein textiles Material oder ein anderes festes, nicht-partikuläres Material handelt, das für Gase oder Flüssigkeiten durchlässig ist, so kann die Einarbeitung beispielsweise erfolgen, indem die Polyphosphatpartikel in das Material eingeblasen werden, etwa mittels eines Luftstroms, oder indem das Material mit einer die Partikel enthaltenen Suspension behandelt wird. Beispielsweise kann man durch Eintauchen eines Vlieses in eine agitierte wässrige Suspension der Partikel, anschließendem Entfernen der wässrigen Phase und Trocknen des Vlieses ein weitgehend gleichmäßig mit Polyphosphatpartikeln beladenes Vlies erhalten, das als erfindungsgemäßes Mittel zur Wundbehandlung geeignet ist.

Zur Behandlung einer blutenden Wunde wird das erfindungsgemäße Mittel im Wundbereich in der Regel im Wundbereich so appliziert, dass die Wunde bzw. der Wundbereich wenigstens teilweise, vorzugsweise vollständig oder zum größten Teil mit dem Mittel bedeckt wird. Nach der Applikation befindet sich das Trägermaterial vorzugsweise in unmittelbarer Nähe zum bzw. im Kontakt mit dem Wundgewebe. Demnach ist das Trägermaterial gemäß einer bevorzugten Ausführungsform in der Lage, sich der Form der Wunde in gewissem Maß anzupassen bzw. weist bereits vor der Applikation in etwa die Form der Wunde auf. Der Fachmann ist daher problemlos in der Lage für eine Vielzahl verschiedenster Wunden das jeweils beste erfindungsgemäße Mittel zur blutstillenden Behandlung auszuwählen, indem er für die jeweilige Wunde ein geeignetes Trägermaterial oder eine geeignete Kombination von Trägermaterialien, identifiziert.

Die erfindungsmäßigen kristallinen Polyphosphate und Mittel zur Wundbehandlung, die solche Polyphosphate enthalten, zeichnen sich durch ausgezeichnete blutstillende Wirkung aus. Außerdem lassen sie sich kostengünstig herstellen, sind leicht zu handhaben, eignen sich für die Behandlung verschiedenster blutender Wunden und sind physiologisch gut verträglich.

Die erfindungsgemäße Verwendungen und Mittel werden durch die nachfolgenden Abbildungen und Beispiele näher erläutert.

### Abbildungen

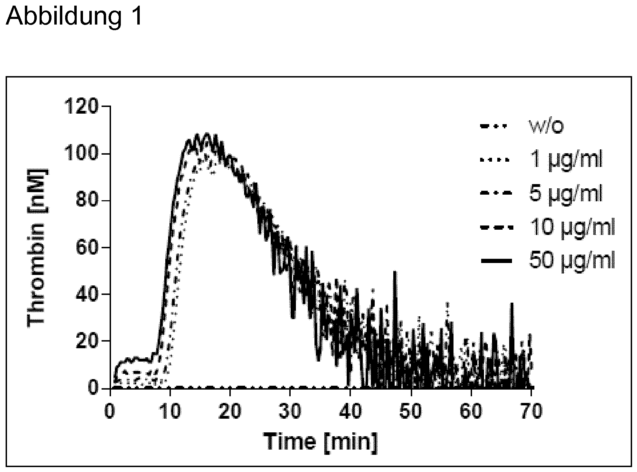

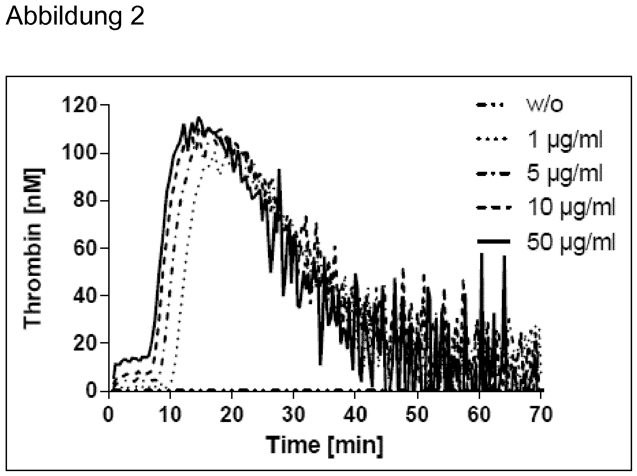

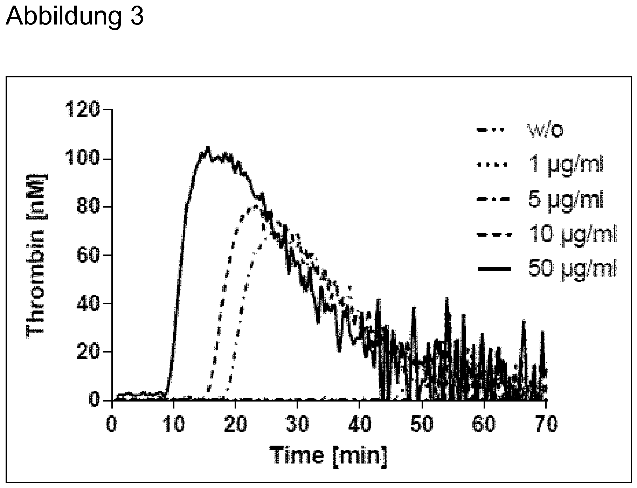

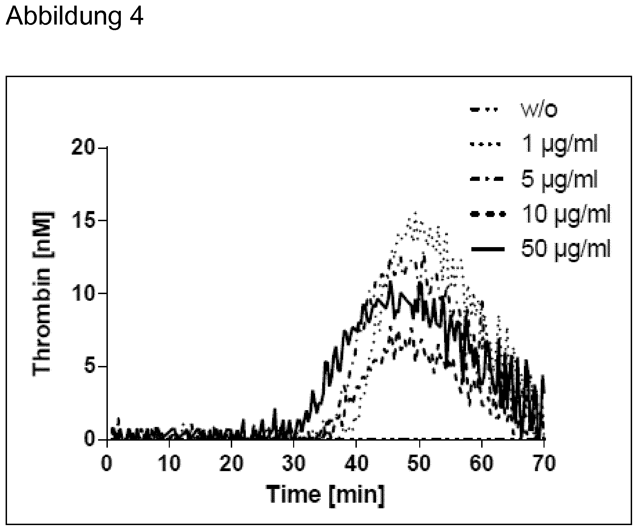

Die Abbildungen 1 bis 4 zeigen Diagramme, die die mit dem hierin beschriebenen Assay zur Echtzeitmessung der Thrombinbildung ermittelten Thrombinkonzentrationen in Blutplasma nach Zugabe von Polyphophat in Abhängigkeit von der Zeit darstellen (sogenannte Thrombogramme). Die Abkürzung "w/o" bedeutet jeweils, dass kein Polyphosphat zugegeben wurde. Die ohne Polyphosphat gemessenen Thrombogramme (also die w/o-Linien) liegen jeweils auf der x-Achse des Diagramms.
Abbildung 1: Thrombogramm ermittelt in Gegenwart von 0, 1, 5, 10 und 50 µg/ml kristallinem Ammoniumpolyphosphat 1 (Kettenlänge K: 38).
Abbildung 2: Thrombogramm ermittelt in Gegenwart von 0, 1, 5, 10 und 50 µg/ml kristallinem Kaliumpolyphosphat (Kettenlänge K: 31).
Abbildung 3: Thrombogramm ermittelt in Gegenwart von 0, 1, 5, 10 und 50 µg/ml kristallinem Natriumpolyphosphat (Kettenlänge K: 44).
Abbildung 4: Thrombogramm ermittelt in Gegenwart von 0, 1, 5, 10 und 50 µg/ml amorphen, wasserlöslichem Natriumpolyphosphat 3 (Kettenlänge K: ca. 27).

### Beispiele

### Bestimmung der Löslichkeit:

Die zu untersuchenden Polyphosphate wurden jeweils in einen 250 ml Erlenmeyerkolben eingewogen (5 g ± 0,01 g) und mit destilliertem Wasser (50 ml, 20-22°C) versetzt. Das so erhaltene Gemisch wurde 20 min bei 200 Umdrehungen pro Minute (UpM, kreisende Bewegung) geschüttelt. Danach wurde die Mischung in ein Zentrifugenglas umgefüllt und 20 min bei 5000 UpM zentrifugiert. Es wurden 10 ml der überstehenden Lösung in ein vorgetrocknetes Aluminiumschälchen (Durchmesser 60 mm, Höhe 15 mm) pipettiert, welches dann bei 105°C im Umluftschrank 150 min getrocknet wurde. Die Löslichkeit in g pro 100g Wasser ergab sich danach aus der Formel TR x 10, wobei TR für den ermittelten Trocknungsrückstand in Gramm steht.

### Bestimmung der Kettenlänge von Polyphosphaten:

Für die verwendeten Polyphosphate wurde ein ³¹P-Festkörper-NMR-Spektrum aufgenommen und die Integrale der Signale der mittelständigen Phosphatgruppen (a) also auch die Integrale der Signale der endständigen Phosphatgruppen (b) ermittelt. Die zahlenmittlere Kettenlänge K ließ sich dann jeweils mit Hilfe der Formel K = 2(a/b +1) bestimmen. Für einige der als Vergleich dienenden wasserlöslichen Polyphosphate wurden die zahlenmittlere Kettenlänge K entsprechend auch mittels ³¹P-NMR in Lösung (D₂O ermittelt. Die erhaltenen Werte für die Kettenlängen K der untersuchten Polyphosphate sind in Tabelle 1 gelistet.

### Bestimmung des Kristallinitätsgrades von Polyphosphaten

Die Kristallinitätsgrade der untersuchten Polyphosphate wurden anhand ihrer Röntgenpulverdiffraktogramme ermittelt. Dabei wurden die Halbwertsbreiten der aufgezeichneten Signale der Diffraktogramme in an sich bekannter Weise zur Quantifizierung der Kristallinität herangezogen.

Es wurden die folgenden Polyphosphate eingesetzt:
(1) Natriumtriphosphat: Natriumtripolyphosphat 1331 (BK Giulini);
(2) Natriumoligophosphat: Natriumpolyphosphat P60 (BK Giulini);
(3) Natriumpolyphosphat A: Natriumpolyphosphat P64 (BK Giulini);
(4) Natriumpolyphosphat B: Natriumpolyphosphat P68 Hexametaphosphat (BK Giulini);
(5) Natriumpolyphosphat C: Natriumpolyphosphat P70 (BK Giulini);
(6) Natriumpolyphosphat D: saures Natriumpolyphosphat P70,5 (BK Giulini);
(7) Natriumpolyphosphat E: Maddrell'sches Salz (BK Giulini);
(8) Ammoniumpolyphosphat A: Phos-Chek P30 (BK Giulini);
(9) Ammoniumpolyphosphat B: Phos-Chek P42 (BK Giulini);
(10) Calciumpolyphosphat: Laborprodukt von BK Giulini;
(11) Kaliumpolyphosphat: Kaliummetaphosphat (BK Giulini);
(12) Natriumtrimetaphosphat: Natriumtrimetaphosphat (BK Giulini);
(13) Calciumhydrogenphosphat: DCP-D, Dicalciumphophat Dihydrat (BK Giulini);

### Assay zur Messung der Thrombinbildung in Echtzeit

Mit diesem Verfahren wurde die durch die Polyphosphate jeweils ausgelöste Bildung von Thrombin im Blutplasma in Abhängigkeit von der Zeit und den eingesetzten Mengen an Polyphosphat ermittelt. Mit Hilfe der auf diese Weise erhaltenen sogenannten Thrombogramme lassen sich Rückschlüsse auf die Ansprechgeschwindigkeit und den Umfang der Thrombinbildung ziehen. Die Messungen wurden entsprechend der Anleitung von Thrombinoscope BV (Hersteller des unten genannten fluorogenen Substrats) in einem Fluorometer (Fluoroscan Ascent, Thermo Scientific), das mit einem Dispenser ausgerüstet ist, durchgeführt (siehe F. Müller et al., Cell 2009, 139, 1143-1156 und Supplemental Data). Die Thrombinbildung erfolgte dabei in einem Gesamtvolumen von 120 µl, das Thrombozyten-armes Plasma (80 µl), Gewebsfaktor (0,5 pM oder 0,0 pM), am aktiven Zentrum inhibierten Faktor Vlla, Phospholipid (4 µM) bestehend aus Phosphatidylserine, Phosphatidylethanolamine und Phosphatidylcholine im Verhältnis 20 mol-% / 20 mol-% / 60 mol-%, Ca²⁺ (20 mM) und das fluorogene Substrat Z-Gly-Gly-Arg-7-amino-4-methylcoumarin (2,5 mM, ZGGR-AMC, Thrombinoscope BV) enthielt. Trypsin-Inhibitor aus Mais, monoklonale Antikörper gegen humanen "Tissue factor pathway inhibitor" (TFPI) oder "Thrombin activatable fibrinolysis inhibitor" (TAFI) und Carboxypeptidase-Inhibitor (alle Sigma-Aldrich GmbH) wurden bis zu Endkonzentrationen von 40 µg/ml, 1 µg/ml bzw. 10 µg/ml zugegeben. Zur Korrektur von inneren Filtereffekten und des Substratverbrauchs wurde jede Messung gegen eine Fluoreszenzkurve kalibriert, die mittels eines Gemisches des Plasmas (80 µl) mit einer festen Menge an Thrombin-α2-Makroglobulin-Komplex (Thrombin Calibrator, Thrombinoscope BV) ermittelt wurde. Alle Messungen wurden jeweils 60 min laufen gelassen und doppelt durchgeführt. Die erhaltenen Werte zur Thrombinbildung wurden mit der Software von Thrombinoscope (Version 3.0.0.29) kalibriert. Die ermittelten Daten für Ammoniumpolyphosphat 1 (Kettenlänge K: 38), Kaliumpolyphosphat (Kettenlänge K: 31), Natriumpolyphosphat (Kettenlänge K: 44) sowie wasserlöslichem Natriumpolyphosphat 3 (Kettenlänge K: ca. 27) als Vergleichsbeispiel sind in den Thrombogrammen der Abbildungen 1 bis 4 zusammengefasst.

Die Thrombogramme werden insbesondere durch die Verzögerungszeit und das endogene Thrombinpotential (ETP) charakterisiert. Bei der Verzögerungszeit handelt es sich um die Zeitspanne vom Zeitpunkt 0 bis zum Beginn des explosionsartigen Anstiegs der Thrombinkonzentration. Als ETP wird das Integral der gemessenen Kurve der Thrombinkonzentration bezeichnet. Die Verzögerungszeit stimmt in erster Näherung mit der Zeit bis zum Einsetzen der Blutgerinnung überein, während das ETP ein guter Indikator für das Ausmaß der Blutgerinnung ist (vergl. Hemker et al., Thromb. Haemost. 2006, 96 (55), 553-561).

Der Vergleich der Thrombogramme für die erfindungsgemäßen kristallinen Polyphosphate mit dem Thrombogramm des wasserlöslichen Natriumpolyphosphats 3 zeigt, dass die erfindungsgemäßen Polyphosphate eine um etwa 3- bis 5-mal kürzere Nachlaufzeit haben und zudem ETPs aufweisen, die um ein Vielfaches größer sind. Daraus lässt sich schließen, dass gegenüber dem wasserlöslichen Natriumpolyphosphat 3 die wasserunlöslichen, kristallinen Polyphosphate der Erfindung nicht nur eine drastisch verkürzte Zeitspanne bis zum Einsetzen der Blutgerinnung zur Folge haben, sondern auch zu einem enormen Anstieg der Thrombinkonzentration und damit zu einer sehr viel intensiveren Blutgerinnung führen.

In der im Folgenden aufgeführten Tabelle 1 werden die untersuchten Phosphate hinsichtlich ihrer Wasserlöslichkeit, ihrer Kettenlänge, ihrer Feststoffstruktur und ihrer blutstillenden Wirkung charakterisiert. Als wasserlöslich sind hier Phosphate gekennzeichnet, die eine mit der oben beschriebenen Methode bestimmte Wasserlöslichkeit von mindestens 1 g pro 100g Wasser aufweisen. Als blutstillend sind hier Phosphate gekennzeichnet, deren nach der oben beschriebenen Methode ermittelten Thrombogramme eine maximale Thrombinkonzentration von mindestens 50 nM aufweisen. Wie sich eindeutig aus der Tabelle 1 ablesen lässt, zeichnen sich alle kristallinen, wasserunlöslichen Polyphosphate gemäß der Erfindung durch eine blutstillende Wirkung aus, wohingegen amorphe, wasserlösliche Polyphosphate sowie kristalline Mono-, Tri- und Metaphosphate nicht blutstillend sind.

**Tabelle 1: Blutstillende Wirkung der erfindungsgemäßen Polyphosphate gemäß Beispielen 1 bis 5 und der nicht erfindungsgemäßen Phosphate bzw. Polyphosphate gemäß Beispielen V1 bis V8.**

| Beispiel | Untersuchte Phosphate | Struktur | Kettenlänge K per ³¹P-NMR in: | | wasserlöslich | blutstillend |
|---|---|---|---|---|---|---|
| | | | fester Phase | Lösung | | |
| V1 | Natriumtriphosphat | kristallin | 3 | 3 | ja | nein |
| V2 | Natriumoligophosphat | amorph | - | 5 | ja | nein |
| V3 | Natriumpolyphosphat A | amorph | - | 7 | ja | nein |
| V4 | Natriumpolyphosphat B | amorph | - | 15 | ja | nein |
| V5 | Natriumpolyphosphat C | amorph | ca. 27 | 31 | 99% | nein |
| V6 | Natriumpolyphosphat D | amorph | - | - | 98% | nein |
| 1 | Natriumpolyphosphat E | kristallin | 44 | - | nein | ja |
| 2 | Ammoniumpolyphosphat A | kristallin | 38 | - | nein | ja |
| 3 | Ammoniumpolyphosphat B | kristallin | 110 | - | nein | ja |
| 4 | Calciumpolyphosphat | kristallin | 16 | - | nein | ja |
| 5 | Kaliumpolyphosphat | kristallin | 31 | - | nein | ja |
| V7 | Natriumtrimetaphosphat | kristallin | dreigliedriger Ring | - | ja | nein |
| V8 | Calciumhydrogenphosphat | kristallin | 1 | - | nein | nein |

## Patentansprüche

1. Polyphosphat in kristalliner Form zur Verwendung in der Behandlung von Wunden, insbesondere blutender Wunden, wobei das Anion des Polyphosphats im Mittel (Zahlenmittel) mindestens 4 Phosphoratome pro Polyphosphat-Anion aufweist.

2. Polyphosphat in kristalliner Form zur Verwendung gemäß Anspruch 1, wobei das Polyphosphat einen Kristallinitätsgrad, bestimmt durch Röntgendiffraktometrie, von mindestens 90%, insbesondere mindestens 95%, aufweist.

3. Polyphosphat in kristalliner Form zur Verwendung gemäß Anspruch 1 oder Anspruch 2, wobei das Anion des Polyphosphats im wesentlichen linear ist.

4. Polyphosphat in kristalliner Form zur Verwendung gemäß einer der Ansprüche 1 bis 3, wobei das Polyphosphat-Anion im Mittel 8 bis 500, vorzugsweise 12 bis 300 und insbesondere 15 bis 150 Phosphoratome aufweist.

5. Polyphosphat in kristalliner Form zur Verwendung gemäß einer der Ansprüche 1 bis 4, wobei die Kationen des Polyphosphats unabhängig voneinander unter Na⁺, K⁺, NH₄⁺, Ca²⁺, Mg²⁺, Zn²⁺, Sn²⁺, Fe³⁺ und Al³⁺, insbesondere unter Na⁺, K⁺, NH₄⁺ und Ca²⁺, ausgewählt sind.

6. Polyphosphat in kristalliner Form zur Verwendung gemäß einer der Ansprüche 1 bis 5, wobei alle Kationen des Polyphosphats unter Na⁺, K⁺, NH₄⁺ und Ca²⁺, insbesondere unter Na⁺, K⁺ und NH₄⁺ ausgewählt sind.

7. Polyphosphat in kristalliner Form zur Verwendung gemäß einer der Ansprüche 1 bis 6, wobei die Partikel des kristallinien Polyphosphats eine gewichtsmittlere Teilchengröße im Bereich von 1 µm bis 1 mm, insbesondere von 1 µm bis 0,5 mm aufweisen.

8. Mittel zur Behandlung von Wunden umfassend ein wie in einem der Ansprüche 1 bis 7 definiertes kristallines Polyphosphat und ein Trägermaterial.

9. Mittel gemäß Anspruch 8, enthaltend zusätzlich wenigstens ein weiteres blutstillendes Agens, das insbesondere ausgewählt ist unter Chitosan, dessen Derivaten, Thrombin, Fibrin, Fibrinogen und Ca²⁺-Ionen enthaltenden Substanzen, die von kristallinem Calciumpolyphosphat verschieden sind.

10. Mittel gemäß einer der Ansprüche 8 oder 9, wobei das Trägermaterial ein textiles Material, eine Flüssigkeit, ein Gel, ein Pulver oder eine Kombination daraus ist.

11. Mittel gemäß einer der Ansprüche 8 bis 10, wobei das kristalline Polyphosphat in partikulärer Form im Trägermaterial verteilt vorliegt.

12. Mittel gemäß einer der Ansprüche 8 bis 11 in Form einer Wundauflage, eines Verbandmaterials, eines Wundpuders, eines Wundssprays, eines Wundschaums, eines Wundgels, einer Wundcreme oder einer Wundpaste.

13. Mittel gemäß Anspruch 11, wobei das kristalline Polyphosphat in partikulärer Form zwischen zwei Lagen eines flächenförmigen, blutdurchlässigen Trägermaterials, insbesondere eines Gewebes oder eines Vlieses, eingeschlossen ist.

14. Mittel gemäß einer der Ansprüche 8 bis 13, das steril ist und gegebenenfalls verpackt ist.

15. Verfahren zur Herstellung des Mittels gemäß einer der Ansprüche 8 bis 14, umfassend das Einarbeiten des Polyphosphats und gegebenenfalls weiterer blutstillender Agenzien in das Trägermaterial, in eine Komponente oder in eine Vorstufe davon.

## Claims

1. Polyphosphate in crystalline form for use in treating wounds, more particularly bleeding wounds, wherein the anion of the polyphosphate has on average (number average) at least 4 phosphorus atoms per polyphosphate anion.

2. Polyphosphate in crystalline form for use as claimed in claim 1, wherein the polyphosphate has a degree of crystallinity, determined by X-ray diffractometry, of at least 90%, more particularly at least 95%.

3. Polyphosphate in crystalline form for use as claimed in claim 1 or claim 2, wherein the anion of the polyphosphate is substantially linear.

4. Polyphosphate in crystalline form for use as claimed in any of claims 1 to 3, wherein the polyphosphate anion has on average from 8 to 500, preferably from 12 to 300 and more particularly from 15 to 150 phosphorus atoms.

5. Polyphosphate in crystalline form for use as claimed in any of claims 1 to 4, wherein the cations of the polyphosphate are independently selected from Na⁺, K⁺, NH₄⁺, Ca²⁺, Mg²⁺, Zn²⁺, Sn²⁺, Fe³⁺ and Al³⁺, more particularly from Na⁺, K⁺, NH₄⁺ and Ca²+.

6. Polyphosphate in crystalline form for use as claimed in any of claims 1 to 5, wherein all cations of the polyphosphate are selected from Na⁺, K⁺, NH₄⁺ and Ca²⁺, more particularly from Na⁺, K⁺ and NH₄⁺.

7. Polyphosphate in crystalline form for use as claimed in any of claims 1 to 6, wherein the particles of the crystalline polyphosphate have a weight-average particle size within the range of 1 µm to 1 mm, more particularly of 1 µm to 0.5 mm.

8. A composition for treating wounds, comprising a crystalline polyphosphate as defined in any of claims 1 to 7 and a carrier material.

9. The composition as claimed in claim 8, additionally containing at least one further hemostatic agent especially selected from chitosan, the derivatives thereof, thrombin, fibrin, fibrinogen and Ca²⁺-ion-containing substances different from crystalline calcium polyphosphate.

10. The composition as claimed in either of claims 8 and 9, wherein the carrier material is a textile material, a liquid, a gel, a powder or a combination thereof.

11. The composition as claimed in any of claims 8 to 10, wherein the crystalline polyphosphate is present distributed in particulate form in the carrier material.

12. The composition as claimed in any of claims 8 to 11 in the form of a wound dressing, a bandaging material, a wound powder, a wound spray, a wound foam, a wound gel, a wound cream or a wound paste.

13. The composition as claimed in claim 11, wherein the crystalline polyphosphate in particulate form is enclosed between two layers of a planar, blood-permeable carrier material, more particularly a woven or a nonwoven.

14. The composition as claimed in any of claims 8 to 13, which is sterile and is optionally packaged.

15. A method for preparing the composition according to any of claims 8 to 14, comprising incorporating the polyphosphate and optionally further hemostatic agents into the carrier material, into a component thereof or into a precursor thereof.

## Revendications

1. Polyphosphate sous forme cristalline destiné à une utilisation dans le traitement de plaies, notamment de plaies saignantes, dans lequel l'anion du polyphosphate comprend en moyenne (moyenne en nombre) au moins 4 atomes de phosphore par anion du polyphosphate.

2. Polyphosphate sous forme cristalline destiné à une utilisation selon la revendication 1, dans lequel le polyphosphate présente un degré de cristallinité, déterminé par diffractométrie de rayons X, d'au moins 90 %, notamment d'au moins 95 %.

3. Polyphosphate sous forme cristalline destiné à une utilisation selon la revendication 1 ou la revendication 2, dans lequel l'anion du polyphosphate est essentiellement linéaire.

4. Polyphosphate sous forme cristalline destiné à une utilisation selon l'une quelconque des revendications 1 à 3, dans lequel l'anion du polyphosphate comprend en moyenne 8 à 500, de préférence 12 à 300 et notamment 15 à 150 atomes de phosphore.

5. Polyphosphate sous forme cristalline destiné à une utilisation selon l'une quelconque des revendications 1 à 4, dans lequel les cations du polyphosphate sont choisis indépendamment les uns des autres parmi Na⁺, K⁺, NH₄⁺, Ca²⁺, Mg²⁺, Zn²⁺, Sn²⁺, Fe³⁺ et Al³⁺, notamment parmi Na⁺, K⁺, NH₄⁺ et Cₐ²⁺.

6. Polyphosphate sous forme cristalline destiné à une utilisation selon l'une quelconque des revendications 1 à 5, dans lequel tous les cations du polyphosphate sont choisis parmi Na⁺, K⁺, NH₄⁺ et Ca²⁺, notamment parmi Na⁺, K⁺ et NH₄⁺.

7. Polyphosphate sous forme cristalline destiné à une utilisation selon l'une quelconque des revendications 1 à 6, dans lequel les particules du polyphosphate cristallin présentent une taille de particule moyenne en poids dans la plage allant de 1 µm à 1 mm, notamment de 1 µm à 0,5 mm.

8. Moyen pour le traitement de plaies comprenant un polyphosphate cristallin tel que défini dans l'une quelconque des revendications 1 à 7 et un matériau support.

9. Moyen selon la revendication 8, contenant en outre au moins un agent hémostatique supplémentaire, qui est notamment choisi parmi le chitosan, ses dérivés, la thrombine, la fibrine, le fibrinogène et les substances contenant des ions Ca²⁺ qui sont différentes du polyphosphate de calcium cristallin.

10. Moyen selon l'une quelconque des revendications 8 ou 9, dans lequel le matériau support est un matériau textile, un liquide, un gel, une poudre ou une combinaison d'entre eux.

11. Moyen selon l'une quelconque des revendications 8 à 10, dans lequel le polyphosphate cristallin se présente réparti sous forme particulaire dans le matériau support.

12. Moyen selon l'une quelconque des revendications 8 à 11, sous la forme d'un pansement pour plaies, d'un matériau de bandage, d'une poudre pour plaies, d'une pulvérisation pour plaies, d'une mousse pour plaies, d'un gel pour plaies, d'une crème pour plaies ou d'une pâte pour plaies.

13. Moyen selon la revendication 11, dans lequel le polyphosphate cristallin est inclus sous forme particulaire entre deux couches d'un matériau support plat perméable au sang, notamment d'un tissu ou d'un non-tissé.

14. Moyen selon l'une quelconque des revendications 8 à 13, qui est stérile et qui est éventuellement emballé.

15. Procédé de fabrication du moyen selon l'une quelconque des revendications 8 à 14, comprenant l'incorporation du polyphosphate et éventuellement d'autres agents hémostatiques dans le matériau support, dans un composant ou dans un précurseur de celui-ci.
